# EUROPEAN PATENT APPLICATION

(11) **EP 1 894 559 A1**
(43) Date of publication of application: **05.03.2008**
(21) Application number: 06018356.3
(22) Date of filing: 01.09.2006
(51) Int. Cl.: A61K 9/10, A61K 9/12, A61K 47/48, A61K 31/57, A61K 31/58

(54) **Means to solubilise steroids for inhalation**

(71) Applicant: PARI Pharma GmbH, 82319 Starnberg (DE)
(72) Inventor: Keller, Manfred, 81379 München (DE)
(74) Representative: Beckmann, Claus

(57) **Abstract**

Sterile compositions for administration as aerosols are described. They contain an active agent which is a poorly water-soluble corticosteroid, preferably budesonide, fluticasone, mometasone, or ciclesonide, and a cyclodextrin. The compositions are suitable for oral or nasal inhalation. They are particularly useful for the pulmonary administration of corticosteroids using common jet or electronic nebulisers.

## Description

### Technical field of the invention

The invention relates to compositions which are useful as medicaments, or components of medicaments, for inhalation in aerosol form. The compositions contain an active agent and can be used pharmaceutically or to promote general well-being. In particular, the invention relates to sterile aqueous preparations for inhalation using a pump spray, a nebuliser, such as a jet, ultrasonic, or vibrating membrane nebuliser, or another aerosol generation system suitable for aqueous liquids. It further relates to the nasal or pulmonary administration of physicochemically, organoleptically or physiologically problematic active agents. According to further embodiments, the invention relates to methods for the manufacture of such compositions and their uses.

### Background of the invention

The treatment of lung diseases by means of aerosols allows a targeted pharmaceutical therapy because the active agent can be delivered directly to the pharmacological target site by means of inhalation devices [D. Köhler and W. Fleischer: Theorie und Praxis der Inhalationstherapie, Arcis Verlag GmbH, München, 2000, ISBN 3-89075-140-7]. This requires that the inhaled droplets or particles reach the target tissue and are deposited there. The smaller the diameter of the aerosol particles, the greater is the probability that active agents reach the peripheral parts of the lungs. Depending on the kind and extent of the deposition, diseases such as asthma, chronic obstructive pulmonary disease (COPD) or pulmonary emphysema can be treated quasi-topically by inhalation. Moreover, systemically active agents such as insulin can be administered to the lung and taken up into the blood circulation by predominantly alveolar absorption. At present, mainly pressurized gas propelled metered dose inhalers, powder inhalers and nebulisers are used for the administration of active agents by inhalation. The type and extent of deposition at the target site depends on the droplet or particle size, the anatomy of the respiratory tract of humans or animals and on the breathing pattern. For the deposition of aerosols in the lungs of rodents such as rats, much smaller droplets are required than, for example, for horses, due to the smaller dimensions of the respiratory tract.

For pulmonary deposition in adults, aerosol droplets or particles should have an aerodynamic diameter of less than 5 - 6 µm, and for infants less than 2 - 3 µm. Moreover, infants breath through the nose, which is why nebulising systems with a nasal mask should be used for the administration of active agents by inhalation. This restriction also applies in the case of other species such as rodents. The influences on the aerosol generation and deposition are mainly influenced by 3 factors which can be categorized as follows:
(1) bio-physiological factors, which are characterized by:
   - the kind of the breathing manoeuvre such as breathing frequency, flow, rate and volume,
   - the anatomy of the respiratory tract, in particular the glottal region,
   - the age and the state of health of the patient;
(2) the droplet or particle spectrum, which is, in turn, influenced by:
   - the kind and construction of the inhalation device,
   - the time interval between generation and inhalation (drying properties),
   - the modification of the droplet or particle spectrum by the respiratory flow,
   - the stability or integrity of the generated aerosol cloud;
(3) the active agent or active agent preparation, whose properties are influenced by:
   - the particle size,
   - the dosage form (for example, solution, suspension, emulsion, liposome dispersion),
   - the shape and surface properties of the active agent particles or the carrier particles (smooth spheres or folded porous structures) in the case of powder aerosols,
   - the hygroscopicity (influences the growth of the particles),
   - the interfacial properties such as wettability and spreadability,
   - the evaporation properties of the carrier medium.

The advantages and disadvantages of the various inhalation devices and the possibilities to compensate inherent disadvantages have been discussed by M. Keller [Development and Trends in Pulmonary Drug Delivery, Chimica Oggi, Chemistry today, No. 11/12, 1998].

The question where aerosol particles are deposited in the bronchial tree has been the subject of numerous investigations for several years. These investigations are supplemented by constantly improving computational models of pulmonary deposition. The regional deposition pattern in breathing through the mouth shows a high degree of variability due to the breathing manoeuvre and the varying anatomy of the bronchial tree. The respirable size range of 0.5 - 6 µm, which is frequently mentioned in the literature, does not take into account the overlapping regions of deposition nor the quantitative or relative deposition rates.

In a healthy adult breathing through the mouth about 40 - 60 % of the particles in the range of 2.5 - 4.5 µm are preferably deposited in the alveolar region. A bronchial deposition of the order of magnitude of about 5 - 28 % is exhibited for particles of 2 to 8 µm, while the oropharyngeal deposition increases in parallel. For particles of 6 µm, the deposition in the oropharynx already amounts to 25 - 45 % and increases to 60 - 80 % for particles with a diameter of 10 µm. It follows from this that, for an optimal qualitative and quantitative alveolar deposition in adult, particle sizes of 1.5 - 3 µm are advantageous if the oropharyngeal and bronchial deposition is to be as low as possible. The bronchial deposition of about 18 - 28 % for particles in the size range of 6 - 9 µm is relatively low and is always accompanied by a correspondingly higher oropharyngeal deposition. Depending on the state of health, the geometry of the bronchial system and the age of the patient, the orders of magnitude stated above shift to smaller particles sizes, in particular in children and babies. In the case of infants of less than 1 year of age, it is assumed that only droplets or particles with an aerodynamic diameter less than 2 - 3 µm reach the deeper regions of the lungs to a significant extent.

For the treatment of sinusitis it is also known that only the smallest aerosol droplets reach the sinuses through the small ostio openings such that more active agent can be deposited at the target site by means of a pulse aerosol than with continuous nebulisation.

The deposition of aerosol particles in the respiratory act is essentially determined by the following four parameters:
- the particle size,
- the particle velocity,
- the geometry of the respiratory tract, and
- the inhalation technique or breathing manoeuvre.

It can be derived from Stokes' law that the flow velocity and density of aerosol particles are relevant, which is why the aerodynamic and not the geometric particle diameter is used as the quantity to be measured for the deposition behaviour in the respiratory tract. It is known from various investigations that only droplet or particle sizes with an aerodynamic diameter of about 0.6 µm - 6 µm can be employed for pulmonary therapy. Particles with an aerodynamic diameter of greater than about 6 µm impact in the upper respiratory tract, whereas those which are smaller than about 1 µm are exhaled after inhalation. This implies that, for example, powders with very low density and an aerodynamic diameter of about 3 µm can have a geometric diameter of, for example, greater than 10 µm. In aqueous systems, on the contrary, with density of about 1 mg/cm3, the geometric and aerodynamic diameters are approximately equal.

The droplet compositions and form of aerosols are very diverse. Depending on the composition, aerosols may have a short or long life time; their droplet or particle size is subject to changes, which is influenced by the physical-chemical properties of the formulation components. Depending on atmospheric humidity, small aqueous droplets evaporate quickly to give a solid nucleus so that the concentration of the dissolved substance(s) upon complete evaporation is 100 %. The resulting diameter (d₂), starting from the original diameter (d₁) corresponds to the cubic root of the concentration ratio before (c₁) and after (c₂) shrinkage (assuming a density of 1 g/cm³ for the dissolved substance) according to the formula: d₂ = d₁ ³√(c₁/c₂). Thus, for example, the drying of aerosols formed by coastal waves by the wind, in the case of a seawater droplet (c₁ = 3.6 %) of 20 µm, results in a salt particle with a diameter of about 6.7 µm, which has, thus, become respirable. This effect is employed, for example, in liquid nebulisers in order to reduce the particle size through drying effects (for example, heating by means of PARI Therm) or admixing of dry air.

On the contrary, in a humid environment, particles can grow and this growth is particularly dependent on the hygroscopicity of the active and/or auxiliary agent. For example, a dry sodium chloride particle of 0.5 µm requires about 1 second for complete growth, whereas in the case of a 5 µm particle this takes about 10 seconds, which proves that the velocity with respect to particle growth is also size dependent. Solid particles from powder nebulisers and metered dose inhalers can grow up to 4 - 5 times of their initial size since the humidity in the bronchial tree is 95 - 100 %. (D. Köhler and W. Fleischer: Theorie und Praxis der Inhalationstherapie, Arcis Verlag GmbH, München 2000, ISBN 3-89075-140-7.)

For toxicological investigations, rodents and dogs are frequently used. Like infants, rodents breath through the nose, which is why in this case the aerosol should be applied by means of a nasal mask in order to achieve a high pulmonary deposition.

For the treatment of some pulmonary diseases such as asthma one mainly uses corticosteroids, beta-agonists and anticholinergic agents which are transported directly to the site of action by means of metered dose inhalers, powder inhalers and jet, ultrasonic or vibrating membrane nebulisers. The pulmonary application of corticosteroids for treatment of asthma has proven to be particularly advantageous compared to oral therapy because the underlying inflammatory process can effectively be inhibited with substantially lower active agent doses with marked reduction of adverse side effects. Active agents such as beclomethasone dipropionate (BDP), budesonide (Bud), and fluticasone propionate (FP) are mainly used as pump sprays for the treatment of allergic diseases in the nasal region, whereas metered dose inhalers (MDI), dry powder inhalers (DPI) and jet nebulisers are used for pulmonary application.

For the therapy of children under the age of 5 years powder inhalers are usually not suitable because children are not capable to generate flows of breath with which powders can reproducibly be de-agglomerated to give respirable particles and deposited in the lungs with sufficient dosage precision. Metered dose inhalers, on the other hand, have the disadvantage that the aerosol is released with a velocity of up to 100 km/h after operation of the valve. Due to insufficient coordination between the triggering of the spray pulse and inhalation, more than 90 % of the active agent impact in the pharynx, which may result in unwanted side effect (hoarseness, voice changes, thrush, etc.). Moreover, the evaporation of the propellant gas can cause a cooling irritation, which, in hyper-reactive patients can result in a de-generation of the epiglottis or in an asthmatic attack, for which reason the inhalation of steroids should always take place with so called spacers with a volume of about 250 - 750 ml. For the application of steroids in infants, who cannot breath through the mouth, there are special types of spacers (for example Babyhaler®) for nasal breathing. However, the use of MDIs and spacers is very complex because active agents sediment, adsorb to the spacer walls or become electrically charged. This can result in insufficient dosage precision and in a non-reproducible pharmaceutical therapy. This is the reason why the nebulisation of aqueous preparations by means of jet, membrane or ultrasonic nebulisers for the pulmonary application of active agents in children and infants is advantageous compared to metered dose inhalers and powder inhalers if sufficiently small droplets or particles are generated.

The ideal situation for a therapy by means of nebulisation of an aqueous preparation is a pharmaceutical substance which is sufficiently soluble and stable in water or isotonic saline solution and whose physical and chemical characteristics do not change during manufacture and storage. If, however, the solubility of the pharmaceutical substance is too low to prepare an aqueous solution of sufficient concentration, nebulisation in the form of a suspension may be considered. By means of a breathing simulator various nebulisation efficiencies (deposited dose, fraction of the pharmaceutical substance re-maining in the nebuliser, etc.) can be detected for the selected pharmaceutical form (suspension or solution). The respirable fraction of the generated aerosol can be determined by measuring the relative proportion of the active agent containing droplets having a geometric or aerodynamic diameter of less than 5 or 3 µm by means of laser diffraction or impactor measurement [N. Luangkhot et al.; Characterization of salbutamol solution compared to budesonide suspensions consisting of submicron and micrometer particles in the PARI LC STAR and a new PARI Electronic Nebuliser (eFlow™). Drug Delivery to the Lungs XI, 11 & 12.12.2000, p. 14-17].

In the aforementioned investigation, it is reported that budesonide-containing suspensions in which the particle size of the suspended pharmaceutical substance is markedly smaller than 1 µm, unlike a microsuspension, can be nebulised with an efficiency similar to that of a salbutamol sulfate solution. This finding is confirmed by Keller et al. [Nebuliser Nanosuspensions. Important device and formulation interactions, Resp. Drug Delivery VIII, 2002, p. 197 - 206]. Moreover, it is pointed out that microsuspensions should not be nebulised with an ultrasonic nebuliser. In a case of the nebulisation of a budesonide suspension (Pulmicort^{®}) it could be shown by ultra centrifugation that about 4.6 % of the budesonide in Pulmicort® are dissolved or solubilised in molecularly dispersed form and that only this fraction can be aerosolized by an ultrasonic nebuliser.

The aqueous corticosteroid preparations which are currently commercially available are microsuspensions of beclomethasone dipropionate (Clenil^{®}), budesonide (Pulmicort^{®}) and fluticasone propionate (Flixotide^{®} ) i.e., the micronized active agent (about 90 % of the suspended pharmaceutical substance particles are smaller than 5 µm) is present in finely dispersed and stabilized form in water. The smaller the particle size is of the active agent and the smaller the density difference between active agent and dispersing medium, the longer the active agent remains in the suspension, i.e., the slower sedimentation usually takes place. Before the application, the sedimented particles or agglomerates must be redispersed in fine form by shaking of the packaging means in order to ensure that an amount as small as possible of the active agent remains in the container and the nebuliser can be filled with the nominal dose. For this purpose and for improved wetting of the lipophilic active agent surface with water a surfactant or wetting agent is added, which, however, must be inhalation-toxicologically safe in order to avoid unwanted side effects. As an example, Pulmicort^{®} shall be referred to, which is commercially available in three concentrations 0.25, 0.5 mg and 1 mg of budesonide per 2 ml. Budesonide is suspended in saline solution which is buffered with citric acid and sodium citrate and contains polysorbate 80 (=Tween^{®} 80) as a wetting agent. The mean particle size of 3 tested lots of Pulmicort® was greater than specified (about 2.8 - 3.2 µm) and scattered between 3.7 and 4.4 µm. This differing finding may possible due to the method of measurement (laser diffraction), but may also be due to particle growth or particle agglomeration. In a publication by Valhi et al. [In-vitro comparison of Pulmicort Repsules with Clenil® per aerosol in combination with three nebulisers, ERS, Annual Congress Stockholm, Sept. 14-18, 2002], electron-microscopic pictures of Pulmicort^{®} and Clenil^{®} were shown from which it follows that the particles in Clenil^{®} are needle-shaped and mainly greater than 10 µm, whereas the Pulmicort^{®} particles are more rounded and have a diameter in the range of about 1 - 6 µm. A further disadvantage is that the aerosol characteristics of such microsuspension can change during nebulisation. This can be derived, for example, from the increase of the budesonide concentration in the residual non-nebulised Pulmicort^{®} suspension. The explanation for this effect is, inter alia, that greater particles cannot be transported by aerosol droplets which have a smaller diameter and, therefore, remain as residue in the nebuliser. In membrane nebulisers, the course particles are retained by the sieving effect of the membrane generating the aerosol. From an economical point of view, this is disadvantageous.

In-vitro investigations using a Baby Cast SAINT Model (Sophia Anatomical Infant Nose Throat) have shown that, upon use of Pulmicort^{®} and a jet nebuliser, only about 1 % of the nominal amount of active agent could be detected as the pulmonary dose. [Using Infant Deposition Models To Improve Inhaler System Design. Resp. Drug Delivery IX, 2004, p. 221-231]. These findings are partly in accordance with clinical findings by paediatricians who report an insufficient efficacy of the Pulmicort® nebulisation therapy in infants and find the explanation for this in that not enough active agent can be transported into the lungs because both the particles and the droplets are too big for infants.

The therapy of the mucosa in the poorly ventilated sinus cavities of the upper respiratory tract is particularly difficult even with easily handled active agents, and all the more so with poorly soluble active agents. Usually, only a very small fraction of the dose of a suspended active agent in aerosolized form reaches the target tissue.

There are a number of suggestions in prior art as to how poorly water soluble active agents can be solubilized or dissolved. In particular, it has been at-tempted to prepare particulate systems with particle sizes in the nanometre range.

Document DE 101 45 361 A1 describes methods for the preparation of nano-particulate systems and a method for the preparation of a sterile submicron suspension. Although such nanosuspensions represent an improvement over conventional microsuspensions with particle diameters of about 1 - 6 µm for the reasons discussed above, they nevertheless have certain disadvantages because, even in the presence of a stabilizing wetting agent, particle growth due to "Ostwald Ripening" during storage cannot be entirely suppressed. Moreover, under certain storage conditions, dissolved particles may precipitate and, as it were, act as seeding crystals to promote particle growth. However, since particle size is of fundamental significance for therapeutic efficiency, products whose particle size cannot be kept constant during common pharmaceutical storage times of 2 - 3 years must be characterized as critical.

Document WO 00/27376 describes nano-particulate aqueous active agent preparations for the preparation of aerosols with the active agent particles exhibiting sizes of less than 1000 nm, preferably less than 50 nm. These preparations are prepared using surface modifying agents which adsorb to the surface of the active agent particles. Vitamin E-TPGS is proposed as one such agent.

Furthermore, it has been suggested to solubilise poorly soluble active agents using colloidal drug carrier systems based on amphiphilic lipids, such as liposomes or mixed micelles. For example, WO 2005/037246 discloses liquid compositions suitable for administration as aerosols which contain a poorly water-soluble drug substance, a non-ionic surfactant component and a phospholipid component. Depending on the relative concentrations, it is believed that the phospholipid and the non-ionic surfactant may assemble to form various types of colloidal carriers in which the drug substance can be incorporated. However, these systems are difficult and costly to manufacture in sterile form, which is required for inhalation products.

Another approach to solubilise poorly soluble compounds such as budesonide is the use of water-miscible organic solvents such as ethanol or propylene glycol, as is described e.g. in EP-A 794 767. While it is not absolutely impossible to use such solvents in combination with water for inhalation purposes, these excipients are highly problematic for a number of physiological reasons, and particularly undesirable for certain patient populations such as children.

WO 2005/065649 and WO 2005/065435 disclose inhalable formulations of glucocorticoids such as budesonide in which the active compound is complexed with a sulfoalkylether-substituted γ-cyclodextrin. It is proposed that the cyclodextrin acts both as a solubiliser and as a stabiliser for the drug substance. Thus, there remains a need for further means for overcoming the problems associated with the poor aqueous solubility of certain drug substances. In particular, there is a need for further means for solubilising poorly soluble compounds for inhalation therapy, and for creating effective corticoid compositions suitable for nebulisation.

Therefore, it is an object of the invention to provide improved compositions of corticoids which are suitable for aerosol therapy and which at least partially overcome one or more disadvantages of prior art.

It is another object of the invention to provide aqueous corticoid formulations which are safe and effective. A further object is to provide compositions and methods for delivering such corticoids in aerosol form to human patients effectively and conveniently.

Further objects will become obvious on the basis of the following description.

### Summary of the Invention

In a first aspect, the invention provides an aqueous pharmaceutical composition suitable for administration as an aerosol which comprises a poorly soluble corticosteroid and a cyclodextrin. The amount of the cyclodextrin is effective to solubilise the corticosteroid. In particular, a cyclodextrin which is not substituted with a sulfoalkylether group is used to solubilise a corticoid such as budesonide, fluticasone, ciclesonide or mometasone.

It has been found by the inventors that particularly γ-cyclodextrin (GCD), 2-hydroxypropyl-γ-cyclodextrin (HPGCD) and 2-hydroxypropyl-β-cyclodextrin (HPBCD) are not only capable of solubilising these active compounds which are so essential in inhalation therapy, but they enable aqueous formulations whose properties allow highly efficient and convenient aerosol delivery using currently available nebulisers. The compositions are particularly useful for being nebulised with modem electronic nebulisers, such as with devices comprising a vibrating membrane.

In a further aspect, the invention provides a method for delivering a pharmaceutical aerosol to a human. According to the method, an aqueous solution of a poorly soluble corticosteroid and a cyclodextrin in an amount effective to solubilise the corticosteroid is nebulised using a nebuliser which is adapted to nebulise the aqueous solution into an aerosol having a mass median aerodynamic diameter of about 1 to about 5 µm.

Further aspects and embodiments of the invention will be disclosed in more detail in the following description, working examples and patent claims.

### Detailed Description of the Invention

In a first principal aspect, the invention provides an aqueous pharmaceutical composition suitable for administration as an aerosol comprising a poorly soluble corticosteroid and a cyclodextrin. The amount of the cyclodextrin in the composition is selected so as to effectively solubilise at least a substantial portion of the corticosteroid comprised in the composition. Surprisingly it was found that in addition to the solubilisation, a stabilisation of the dissolved corticosteroid in an aqueous medium is possible. Even more surprisingly it was found that beta-agonists, such as formoterol-fumarate and anticholinergic drugs, such as thiotropium-bromide, known to be instable in aqueous systems could be stabilised when formulated as a combination product in the presence of one or two types of cyclodextrin.

As used herein, a pharmaceutical composition is a composition of at least one active ingredient suitable for the diagnosis, prevention, or treatment of a symptom, condition or disease in a human or other animal and at least one pharmaceutically acceptable excipient.

The composition of the invention is aqueous, which means that it is preferably a liquid composition comprising water as the predominant liquid constituent. As the composition is intended for administration in aerosol form, it is further preferred for safety and tolerability reasons that water is the only liquid present in the composition. However, in some cases it may be acceptable even for liquids for inhalation to comprise some amount of other liquids, in particular one or more organic solvents having a relatively low inhalation toxicity such as ethanol, propylene glycol, or glycerol.

The aqueous composition is suitable for administration as an aerosol. In particular, it is designed for oral or nasal inhalation. Oral inhalation is usually preferred if the target region where the aerosol is to be deposited is the lower respiratory tract, such as the bronchi or the alveolar region. In contrast, nasal inhalation through one or both nostrils is usually more suitable if the aerosol is administered for the prevention or treatment of a symptom or disease affecting a region of the upper respiratory tract, such as the nasal mucosa or the paranasal sinuses.

The composition comprises an active ingredient selected from the group of corticosteroids. Those corticosteroids (also referred to as steroids, corticoids, glucocorticoids, or cortisol analogues) which are most suitable for inhalation are typically compounds that are locally active but at the same time exhibit little systemic corticoid activity. Examples of such corticosteroids include beclomethasone, budesonide, flunisolide, fluticasone, ciclesonide, mometasone, or any compounds comprising the active moiety of any of these corticosteroids, such as salts, derivatives, and prodrugs thereof.

According to the invention, the corticosteroid selected for the composition is poorly water-soluble. In this context, a poor solubility means that the compound is poorly soluble in aqueous media at room temperature and relatively neutral pH, for example, at pH 4 to 10, and in particular at pH 4 to 8. Moreover, poorly water-soluble means that at least 30 parts of water or aqueous solvent are required for dissolving one part of active agent. This corresponds to substances which are to be characterised by the commonly used terms "sparingly soluble", "slightly soluble", "very slightly soluble", "practically insoluble" and "insoluble". As used herein, poorly water-soluble and poorly soluble refer to the same property.

Poorly soluble active agents also comprise poorly soluble derivatives or salts of active agents, of which more soluble salts or derivatives may exist. The invention is particularly advantageous for the formulation of active agents which have a saturation solubility in water at room temperature of not more than about 0.1 wt-%. Moreover, active agents of the categories "very slightly soluble", "practically insoluble" and "insoluble" are particularly preferred.

Preferably, the corticosteroid is selected from budesonide, fluticasone, ciclesonide, mometasone, including the respective salts, derivatives, and solvates.

The composition of the invention further comprises a cyclodextrin. The amount or content of the cyclodextrin in the composition is selected so as to solubilise at least a major fraction of the active compound, and most preferably so as to solubilise substantially all of the corticosteroid present in the composition.

Cyclodextrins are oligosaccharides composed of glucopyranose units. The major unsubstituted cyclodextrins are usually prepared by the enzymatic degradation of starch. They are conelike, toroidal molecules with a rigid structure and a central cavity, the size of which varies according to the cyclodextrin type. The internal cavity of cyclodextrins is hydrophobic and capable of accommodating lipophilic molecules in the form of inclusion complexes, thus enabling the solubilisation of poorly soluble drugs in aqueous media. More recently, also other types of complexes involving cyclodextrins were identified.

The three major types of cyclodextrins are α-, β- and γ-cyclodextrins, comprising 6, 7 and 8 glucopyranose units, respectively. In addition, a number of chemically modified cyclodextrins have been developed, mostly motivated by the desire to increase the water solubility and extend their usefulness as solubilising excipients. Examples of such derivatives include in particular 2-hydroxypropyl-β-cyclodextrin (HPBCD), 2-hydroxypropyl-γ-cyclodextrin (HPGCD), sulfobutylether-β-cyclodextrin (SBEBCD), and methyl-β-cyclodextrin (MBCD).

The composition preferably comprises the corticosteroid at a concentration from about 20 µg/ml to about 2 mg/ml. In a further embodiment, the corticosteroid is budesonide and its concentration in the composition is from about 0.1 to about 1 mg/ml, and in particular from about 0.2 to about 0.75 mg/ml. According to a further embodiment, the corticosteroid is fluticason-17-priopionate and its concentration in the composition is from about 100 to about 500 µg/ml, and preferably from about 150 to about 350 µg/ml.

In selecting the concentration of the active compound in the aqueous composition, several factors should be taken into consideration. Firstly, the solubility of the corticosteroid depends on the type and concentration of the cyclodextrin which is used in the formulation. Since it is highly desirable that substantially all of the corticosteroid is solubilised, i.e. dissolved, its concentration should be selected below its maximum solubility in combination with the respective cyclodextrin. Secondly, the volume of the composition which is to be administered as a single dose should allow convenient handling and inhalation within an acceptable period of time, and the corticoid concentration should be selected accordingly. For example, if the composition is to be administered using a common jet nebuliser, the volume of a single dose should preferably be in the range from about 1 to about 5 ml. On the other hand, if the composition is designed for inhalation using an advanced electronic nebuliser based on the vibrating mesh design, such as the PARI eFlow device, which is another highly preferred embodiment of the invention, a relatively small volume of about 0.25 to about 1 ml, such as about 0.5 ml, may be selected due to the minimal dead volume of the device. Accordingly, the drug concentration should be adapted to accommodate a single dose within the selected volume. Typically, a single dose is in the range form about 50 to about 250 µg of corticosteroid, in particular for pulmonary inhalation.

The concentration of the cyclodextrin should be selected taking into account the type of cyclodextrin and the concentration of the active ingredient which is to be achieved. Typically, the cyclodextrin concentration in the composition according to the present invention is in the range from about 1 to about 150 mg/ml, and more preferably from about 5 to about 100 mg/ml. Other preferred concentrations range from about 10 to about 75 mg/ml.

If budesonide is selected as corticosteroid, it is further preferred that the cyclodextrin is selected from GCD and HPBCD. In the case of GCD, a concentration in the range from about 5 to about 30 mg/ml may be selected to solubilise the drug substance at a concentration from about 0.1 to 1 mg/ml, or from about 0.2 to about 0.5 mg/ml. A preferred concentration range for HPBCD is from about 10 to about 100 mg/ml, which is particularly useful to achieve a dissolved budesonide concentration of from about 0.1 to 1 mg/ml, or from about 0.2 to 0.75 mg/ml, respectively.

If the corticosteroid is fluticasone-17-propionate, the cyclodextrin is preferably selected from GCD and HPGCD. For example, GCD at a concentration of about 10 to about 75 mg/ml is useful for solubilising fluticasone-17-propionate to achieve a concentration of about 0.1 to 0.5 mg/ml or about 0.15 to 0.35 mg/ml, respectively.

It is further preferred that the pH of the composition is adjusted to about 3.5 to 7.5, in particular to a value within the range from about 4 to about 7. If budesonide is selected as corticosteroid, a pH from 3.5 to 6, and especially from about 4 to 5 is preferred. If the corticosteroid is fluticasone-17-propionate, the pH may also be selected in the range from about 4.5 to 7.5, and more preferably from about 5 to 7.

If the designated use of the composition is pulmonary administration, the osmolality should not deviate too much from that of physiological fluids, i.e., from about 290 mOsmol/kg. However, in individual cases, a compromise may be desirable to reconcile the pharmaceutical needs on one hand and the physiological requirements on the other hand. In general, an osmolality in the range of above about 200 and below about 500 or 600 mOsmol/kg seems useful, and, in particular, in the range of about 220 or 230 up to about 350 mOsmol/kg may be considered acceptable.

Especially for pulmonary application, the surface tension of the composition of the invention should be adjusted to the range of about 25 to 80 mN/m, and preferably to the range of about 30 to 75 mN/m. In this context it is to be taken into consideration that, in the lowest part of this range, a particularly good spreadability of the preparation in the respiratory tract is to be expected, but that the quality of the aerosol and the efficiency of the nebulisation may be adversely affected. On the other hand, solubilising agents such as cyclodextrins as well as other excipients which may be required in the formulation tend to decrease the surface tensions significantly. Thus, a compromise has to be found in each case depending on the active agent and the intended application. Particularly preferred is a surface tension in the range of about 40 to 75 mN/m or, if achievable, even in the range of 45 to 73 mN/m or 50 to 72 mN/m, respectively.

The dynamic viscosity, which may also be influenced by the selection of the drug substance and particularly by the choice and quantity of excipients, also has a clear influence on the particle size distribution of the aerosol formed by nebulisation and on the efficiency of nebulisation. Preferably, the viscosity should be adjusted to a range of about 0.8 to about 3 mPas. Presently still more preferred is a dynamic viscosity in the range of about 0.8 to about 2.5 mPas or even about 0.9 to about 1.3.

In order to adjust the pH, surface tension, viscosity, osmolality, stability, taste and other properties of the composition, one or more further excipients may be used. For example, the composition may comprise one or more agents selected from pharmaceutically acceptable organic acids, salts of organic acids, inorganic acids, inorganic salts, bases, sugars, sugar alcohols, stabilisers, antioxidants, surfactants, preservatives, and taste masking agents.

In addition to the corticosteroid, the composition may comprise a further active ingredient. This may be advantageous, for example, in asthma therapy when more than one active agent must be inhaled for therapeutic reasons and the application by the patient in a short time is to be made possible. Important combinations of active agents in this connection are, for example, those of a corticoid with a betamimetic or parasympatholytic agent. Further combinations may involve mast cell stabilizers such as cromoglicinic acid or nedocromile including their therapeutically useful salts. Combinations with antibiotics and bronchodilators as well as mucolytics may also be useful. The addition of physiologically safe and well tolerated antioxidants such as tocopheroles, catalase, peroxide dismutase, lycopen is, in principle, useful since these absorb or reduce radical oxygen which is responsible for many inflammatory processes. Also, a combination of two corticosteroids may be useful.

According to one of the embodiments in which the composition comprises more than only one active agent, at least one of these additional active compounds is selected from the group consisting of non-steroidal anti-inflammatory drugs (NSAIDs), leukotriene-antagonists such as roflumilast; or betasympathometics, anticholinergics, local anesthetics, immunomodulators, antiinfectives, angiotension converting enzyme (ACE) inhibitors, cytostatics, formoterol, salmeterole, levalbuterole; thiotropium, oxitropium, ipratropium; lidocaine, prolocaine, mepivacaine, bupivacaine, articaine, ciclosporine, tacrolimus, sirolimus, everolimus, rapamycine, azathioprine; ciprofloxacine, moxidloxacine, azithromycine, clarithromycine, erythromycine, metronidazole, ketoconazole, itraconazole, voriconazole, clotrimazole, bifonazole, fluconazole, amphotericine B, natamycine, nystatine, amikacine, aciclovir, famciclovir, valaciclovir, didanosine, saquinavir, ritonavir, lamivudine, stavudine, zidovudine, ribivarine, captoprile, lisinoprile, perindoprile, trandolaprile, cilazaprile, carmustine, lomustine, taxol, etopside, cisplatin; reduced glutathione, TNF-alpha-antibodies including the pharmaceutically acceptable derivatives of these substances such as, for example, the salts, conjugates, enantiomeres, racemates, epimeres, diastereomeres or complexes of any one of these active agents.

As mentioned above, the composition of the invention is preferably provided and formulated as an aqueous liquid. Alternatively, it may be provided and formulated in dry solid form, for example, if the chemical stability of an incorporated ingredient is not sufficient to allow an acceptable shelf life. In this case, the composition may be a powder or a lyophilisate. This may require different, or additional excipients, such as a bulking agent or a lyoprotectant. In one of the more preferred embodiments, the composition is a lyophilisate which is obtainable by freeze drying a liquid composition which has the properties as discussed herein-above.

Furthermore, the solid formulation is adapted to be reconstituted to an aqueous liquid form, which may be achieved, for instance, by combining the solid composition with a suitable aqueous carrier, such as sterile water (e.g. water for injection), sterile sodium chloride solution, sterile buffer solution etc.

The composition of the invention may itself be considered a medicament, or it may be part of a medicament. As used herein, a medicament is broadly defined as a medicinal or drug product used for prophylactic, diagnostic, or therapeutic purposes. The scope of the term is broader than that of a composition, as it may include, for example, the combination of more than one composition, or the combination of a composition with a device. Provided that a composition is the actual drug product which is delivered to the patient or health care provider, it may also be itself understood as a medicament. On the other hand, a composition would also describe a bulk material, whereas the medicament better describes the individual drug product as it is distributed by the pharmaceutical manufacturer.

Thus, according to another aspect of the invention, a medicament comprising the composition of the invention is provided which is packaged for use by a patient or health care provider. In a preferred embodiment, the medicament - or composition - is packaged in single dose primary packaging means. Preferably, more than 90 % of the content can be dispensed from such packaging with a dispensing uniformity of better (i.e. less) than about 10 % of the labelled dose, or volume.

The volume of a single dose of the medicament is generally in the range of about 0.1 to about 10 ml, but more preferably in the range of about 0.2 to about 5 ml. If budesonide or fluticason-17-propionate is selected as the active agent, and the designated use is inhalation, the volume of a single dose is preferably about 0.25 to about 2 ml. Particularly preferred volumes are about 0.5 and 1.0 ml, respectively, especially if the composition is for pulmonary inhalation using a nebuliser with a small or absent dead volume, such as the PARI eFlow.

The primary packaging means can be glass containers (for example, vials) with closure devices of elastomers such as metal security caps, alternatively they may be plastic vials or blister-like primary packaging systems. An individual primary package may contain a single dose or a multiple thereof. In all cases, however, in which heat sterilization of the preparation is possible and does not result in significant deterioration of quality, it is preferable to use a method which, instead of sterile filtration, comprises the final sterilization of the preparation by a heat sterilization method after filling the primary packing means in accordance with the relevant pharmacopoeia.

Suitable primary packaging of plastic are, for example, polypropylene or polyethylene vials (PP-/PE-vials) and cycloolefine copolymer blisters (COC-blisters). Sealed plastic containers such as PP or PE vials may advantageously be formed, filled and sealed by the blow-fill-seal method, which is well known to the technically qualified person in the field. The thus produced containers are suitable, in particular, for liquid goods with a volume starting from about 0.2 ml.

In a particularly patient-friendly embodiment, they may be formed in a bottle shaped design with a closure which can be removed by twisting or bending allowing a dropwise dosing and entire emptying meeting with a dispensing uniformity as claimed by Pharmacopoeias. The thus formed opening through which the liquid content can be removed, may be designed such that it fits onto a luer connection or luer lock connection. Thus, the opening may have a round shape and a diameter which largely corresponds to the outer diameter of a male luer connection. In this way, a common syringe with luer connection could be connected tightly to the container, for example, in order to take up the contents of the container and transfer it to a nebuliser or in order to mix the content of the container with the content of the syringe and subsequently to add it to a nebuliser. Alternatively, the opening may have a smaller diameter, such as the inner diameter of a male luer connective piece, if the opening is within a protrusion which is connectable with a female luer piece.

As a further alternative, it may be envisaged that the plastic container is designed so that, after removing the closure element, it may be connected essentially tightly with a connection piece intended for the addition of liquid of a correspondingly adapted nebuliser, whereby it is possible to fill the preparation directly into the reservoir of the inhaler.

Depending on the intended product application, the medicament may also be presented in multiple unit dose primary packaging means or containers. Such containers may hold a fill volume of about 2 to about 50 ml or even more. If the use is different from inhalation, such product design may be appropriate and cost effective. If the use is oral or nasal inhalation, the medicament must comprise a preservative to ensure its sterility after the withdrawal of a dose. However, the incorporation of a preservative is less preferred for pulmonary administration due to tolerability reasons.

Plastic containers of the above-mentioned kind are advantageous because they can easily be provided with imprints. Thereby, one can firstly do away with paper labels, which is desirable in order to avoid the migration of components of the adhesive, the paper or the printing ink through the container wall into the preparation. Furthermore, important information may be made available to visually impaired patients by such an imprint. The imprint can contain various information, for example, a lot number, a best before date, a product designation, instructions for use or one or more volume or dose markings. Especially for paediatric patients, where a flexible dosing depending on the age and body size is desirable, a plurality of volume markings can serve to facilitate the removal of the desired dose without further implements, thereby reducing the risk of dosing errors.

One or more primary packaging means may be packaged in one secondary packaging means, such as a cardboard box.

As mentioned, in order to increase the storage stability, it may be advantageous to subject the composition according to the invention described above to freeze drying in order to storage in the solid state. The liquid form required for nebulization can be prepared there from shortly before use by mixing with sterile water. Therefore, a solid preparation is also provided in accordance with the invention, which is obtainable by freeze drying the preparation according to the invention described above. The methods of freeze drying are known, as such, to the skilled person.

If the medicament comprises such solid-state composition, it may be useful to provide a secondary package which is in the form of a kit, and which contains one or more primary packaging containers with the solid composition and also one or more containers filled with a suitable aqueous liquid carrier which is to be used for reconstituting the solid composition and form an aqueous liquid system which can be aerosolized, or which is suitable for nasal or oral inhalation.

In a further aspect, the invention provides a method for preparing the aqueous liquid composition defined hereinabove. The method comprises the steps of (a) providing the ingredients of the composition; (b) combining the ingredients provided in step (a) to form an aqueous liquid composition; (c) sterile filtration of the composition obtained in step (b); and (d) filling the sterile filtered composition obtained in step (c) into sterile containers under aseptic conditions. The steps (a) to (d) are conducted in this sequence.

Optionally, the method may include further steps, and each of the steps (a) to (d) may comprise a number of substeps. For example, it may be useful to first sterilise one or more ingredients in order to provide them in sterile form. In this way, the bioburden is kept relatively low with the effect that the sterile filtration step (c) will be less problematic to design.

Furthermore, it may be useful to conduct step (b) as a series of substeps in which ingredients are combined sequentially. For example, it may be advantageous to first form a cyclodextrin solution by combining the cyclodextrin and water before adding the corticosteroid. Also, it is preferred that the pH is first adjusted to the desired value by adding an appropriate acid, base, and/or buffer salt to the water or to the cyclodextrin solution before the corticosteroid is added.

It is furthermore preferred that step (b) comprises a substep of equilibration. In particular, after at least the water, the cyclodextrin and the corticosteroid have been combined, it is useful to equilibrate the mixture over a period of at least about one hour to allow the formation of an inclusion complex between the cyclodextrin and the corticosteroid. For example, it may be useful to first prepare an aqueous solution of the cyclodextrin. Subsequently, further inactive ingredients, in particular pH-and osmolality-adjusting agents may be dissolved in the cyclodextrin solution. Subsequently, the active compound may be added to the solution. A stirring time of at least about one hour, preferably of several hours, will ensure effective complex formation and equilibration.

The equilibration step, like all other steps, may be conducted at normal conditions, i.e. at room temperature and normal pressure. Alternatively, temperature and/or pressure may be adapted to enhance the dissolution of the poorly soluble corticosteroid, to increase the mobility of the molecules involved in complex formation, or to increase the chemical stability of a hydrolytically labile compound during equilibration. The time for equilibration is influenced by the stirring energy and can be reduced by use of high-shear equipment.

Subsequently, sterile filtration of the resulting aqueous solution is conducted. How to select an appropriate filter and conduct the sterile filtration process is per se known to a person trained in the field. Typically, one or two filtrations through filters having a pore size of 0.22 µm, optionally with a prefilter with a pore size of 0.45 µm, are recommended.

The subsequent filling of the sterile solution into the final containers is performed under aseptic conditions. Pre-sterilised glass vials may be selected as containers. More preferably, sterile plastic vials which are manufactured in-line using a blow-fill-seal process design are used, in particular if the product is packaged as single dose units with a dose volume in the range from about 0.2 to about 5 ml.

In a further principal aspect, the invention provides a method for delivering a pharmaceutical aerosol to a human in need thereof, which method comprises providing an aqueous solution of a poorly soluble corticosteroid and a cyclodextrin in an amount effective to solubilise the corticosteroid; providing a nebuliser adapted to nebulise the aqueous solution into an aerosol having a mass median aerodynamic diameter of about 1 to about 5 µm, and more particularly of about 2 to about 4 µm, and using the nebuliser to nebulise said aqueous solution. In a further embodiment, the mass median diameter is from about 3 to about 4 µm and exhibits a geometric standard deviation from about 1.3 to about 1.7.

The aqueous solution of a poorly soluble corticosteroid and a cyclodextrin in an amount effective to solubilise the corticosteroid has been described in further detail hereinabove. As previously mentioned, the corticosteroid is preferably selected from budesonide, fluticasone, mometasone, ciclesonide, including the salts (such as fluticason-17-propionate), derivatives, and solvates thereof, and incorporated into the solution at a concentration of about 20 to about 2000 µg/ml. The cyclodextrin is preferably 2-hydroxypropyl-β-cyclodextrin, γ-cyclodextrin, or 2-hydroxypropyl-γ-cyclodextrin at a concentration from about 5 to 200 mg/ml.

It has now been found that such aqueous corticosteroid composition is advantageously administered with a nebuliser which is adapted to aerosolise this composition into an aerosol having the specified mass median diameter. For the benefit of achieving a high level of patient convenience and compliance, the nebuliser is further adapted to deliver the aerosol at a total output rate of at least about 150 mg/min. More preferably, the total output rate is at least about 250 mg/min or even 350 mg/min. In another embodiment, the total output rate is at least about 400 mg/ml.

If the volume of a single dose of the aqueous composition is relatively small, such as not more than about 1 ml, it is also preferred to select a nebuliser which has a particularly small dead volume (residual volume), in particular a dead volume of less than about 0.3 ml, and preferably one of less than about 0.1 ml.

According to the method of the invention, inhalation therapy with corticosteroids is substantially improved. The particular advantage of using the formulation design of a cyclodextrin-solubilised corticosteroid allows a high active ingredient concentration of the composition, enabling a drug dose to be accommodated in a small volume of liquid. While a small dose volume combined with one of the many conventional nebulisers which have a significant dead volume is less preferred because of the high relative loss of drug in the device, the selection of a nebuliser having the preferred output rate and low dead volume allows very rapid and efficient aerosol inhalation. In particular, it is a preferred feature of the method that the inhalation time is less than about 5 minutes, or even only about 3 minutes or less, or about 2 minutes or less, such as from about 1 to about 2 minutes, or from about 30 to about 60 seconds, respectively. Such short inhalation times are reached by the appropriate selections of the drug dose and drug concentration in solubilised form as discussed above, and the use of one of the preferred nebulisers.

One of the preferred nebuliser types is an electronic nebuliser based on the vibrating mesh design. In this type of device, the aerosol droplets are formed by a vibrating membrane without pores or by extrusion of the inhalation liquid through a vibrating membrane with pores of a defined size. Examples of latter include the eFlow®, eFlow® rapid, AeroNeb®, AeroDose®, AERx®, MicroAir®, and ODEM device. Particularly preferred are the devices of the eFlow® family in the context of the present invention.

These nebulisers are particularly useful if the aerosol is to be orally inhaled for pulmonary delivery, such as to subjects suffering from symptoms or conditions like asthma, paediatric asthma, obstructive bronchitis, chronic obstructive bronchitis, chronic obstructive pulmonary disease (COPD), emphysema, lung infections, vascular, parenchymal lung disease, sarcosidosis, pulmonary fibrosis, cystic fibrosis, bonchiolitis obliterans, pulmonary hypertension, and lung cancer.

Alternatively, the compositions may also be beneficial for subjects being affected by conditions of the upper respiratory system, in particular diseases, symptoms or conditions affecting the mucosa of the nasal cavity and/or a paranasal sinus, such as acute, subacute or chronic forms of allergic and viral rhinitis, vasomotoric rhinitis, seasonal rhinitis, perennial rhinitis, chronic rhinitis, rhinosinusitis, sinusitis, or nasal polyps.

In these cases, it is preferred that the composition is administered in aerosolised form through one or both nostrils of the subject using an appropriately adapted nebuliser. For this use, it is particularly preferred that a nebuliser which emits a vibrating aerosol is selected, such as a PARI Sinus device. One of the key features of a vibrating aerosol is that it pulsates with a selected frequency. As used herein, the pulsation of an aerosol is understood as a periodic change of pressure. Preferably, the pulsation is regular, i.e. the time interval between pressure peaks is approximately constant. The amplitude of pressure pulsation may also be relatively constant, at least with regard to the generation and emission of the pulsating aerosol from the aerosol generator.

Preferably, the pressure of the aerosol pulsates with a frequency in the range from about 10 Hz to about 90 Hz. According to some of the presently preferred embodiments, the pressure may also pulsate at a frequency in the range from about 10 to about 60 Hz, or from 10 to about 55 Hz, or from about 30 to about 60 Hz. In a further embodiment, the aerosol vibrates at a frequency of about 30 to about 55 Hz, such as from about 40 to about 50 Hz, for example about 44 Hz.

It has been found that a vibrating aerosol enters the paranasal sinuses after nasal inhalation to a much larger extent than a conventional aerosol having a substantially constant pressure, provided that the appropriate particles sizes are selected as outlined above. Larger particle sizes will lead to little sinus deposition, but to a large deposition on the nasal mucosa, whereas very small particle sizes allow the aerosol droplets to enter the sinuses following the pressure gradient of a pressure pulse, but also their exit from the sinuses without them being deposited therein.

The principle of generating and applying a pulsating or vibrating aerosol for enhanced sinus deposition has recently been found and described, for example, in EP-A 0 507 707 and WO 2004/020029, whose entire disclosures are incorporated herein by reference.

It is believed that an aerosol flow which is superimposed with pressure fluctuations, or pressure pulses, creates periodic transient pressure gradients extending from the actively ventilated nasal cavity through the ostia to the sinuses, which gradients cause a short period of convective flow of air and aerosol into the sinuses until the pressure therein has become equal to the air pressure in the nasal cavity. A portion of the aerosol droplets which thus enter the paranasal sinuses are deposited therein onto the mucosa. The extent to which the aerosol is deposited depends e.g. on the droplet size. Droplets that are smaller than the preferred particle size are relatively likely to be expelled from the sinuses during the subsequent pulsation phase in which the aerosol pressure, and thus the pressure in the nasal cavity, is lower than the pressure within the sinuses, and during which a convective flow of air from the sinuses to the nasal cavity occurs.

Alternatively, conventional jet nebulisers may also be used, such as the PARI LC Sprint device. In this embodiment, the advantage of short inhalation times is less pronounced. On the other hand, commonly available and cost-effective equipment is used.

The invention is further illustrated by means of the following non-limiting examples.

### Example 1: Preparation of a budesonide solution with HPBCD

5 g of HPBCD were dissolved in 90 g of sterile, purified water (water for injection, WFI) by stirring at room temperature. 0.55 g of sodium chloride, 0.5 g of sodium ascorbate and a sufficient amount of hydrochloric acid (1 M) to adjust the pH to 4.5 were added. 30 mg of budesonide were suspended in the solution, and WFI was added to a weight of 100 g. The liquid was equilibrated by stirring at room temperature for about 5 hours. A clear solution was obtained and filtered through a sterile filter having a pore size of 0.22 µm. 0.5 ml, each of this solution were filled under light protection into polyethylene blow-fill-seal vials. The assay of the resulting solution showed a budesonide content of approx. 286 µg/ml, indicating that all of the active ingredient had been incorporated in solubilised form. The composition had a dynamic viscosity of about 1.24 mPas and a surface tension of approx. 60 mN/m. The osmolality was approx. 295 mOsmol/kg.

### Example 2: Preparation of a budesonide solution with HPBCD

The experiment of example 1 was repeated except that 50 mg of budesonide were used. After sterile filtration (0.22 µm), a clear solution resulted containing approx. 500 µg/ml of budesonide in solubilised form. A volume of 0.75 ml of the sterile filtered solution was filled in blow-fill-seal vials made of low density polyethylene.

### Example 3: Preparation of a budesonide solution with GCD

2 g of GCD were dissolved in 90 g of WFI. 0.9 g of sodium chloride, 0.04 g of citric acid, and 0.05 g of disodium citrate dihydrate were dissolved in the solution. Subsequently, the pH was adjusted to 4.5 by the addition of sodium citrate dihydrate. Next, 40 mg of budesonide and a sufficient amount of WFI to yield a total weight of 100 g were added. The liquid was stirred at room temperature for about 15 hours and filtered through a sterile filter having a pore size of 0.22 µm. The assay of the resulting solution showed a budesonide content of approx. 400 µg/ml, indicating that all of the active ingredient had been incorporated in solubilised form. The composition had a dynamic viscosity of about 1.08 mPas and a surface tension of approx. 73 mN/m.

### Example 4: Preparation of a fluticasone solution with GCD

5 g of GCD were dissolved in 90 g of WFI. To the solution, 0.9 g of sodium chloride and a sufficient amount of sodium hydroxide solution (0.1 M) or hydrochloric acid (0.1 M), respectively, to obtain a pH of 6.0 were added. Subsequently, 20 mg of fluticason-17-propionate and a sufficient amount of WFI to yield a total weight of 100 g were added. The liquid was stirred at room temperature for about 15 hours and filtered through a sterile filter having a pore size of 0.22 µm. The assay of the resulting solution showed a fluticason-17-propionate content of approx. 200 µg/ml. The composition had a dynamic viscosity of about 1.13 mPas and a surface tension of approx. 72 mN/m.

### Example 5: Preparation of a fluticasone solution with HPGCD

10 g of HPGCD were dissolved in 85 g of WFI. To the solution, 0.9 g of sodium chloride and a sufficient amount of sodium hydroxide solution (0.1 M) or hydrochloric acid (0.1 M), respectively, to obtain a pH of 6.0 were added. Subsequently, 30 mg of fluticason-17-propionate and a sufficient amount of WFI to yield a total weight of 100 g were added. The liquid was stirred at room temperature for about 5 hours and filtered through a sterile filter having a pore size of 0.22 µm. The assay of the resulting solution showed a fluticason-17-propionate content of approx. 300 µg/ml. The composition had a dynamic viscosity of about 1.41 mPas and a surface tension of approx. 60 mN/m.

### Example 6: Preparation of a fluticasone and formoterol solution with HPGCD

7 g of HPGCD were dissolved in 90 g of WFI. To the solution, 2.4 mg formoterol fumarate dihydrate (equals 2 mg Formoterol), 0.9 g of sodium chloride and a sufficient amount of hydrochloric acid (0.1 M) to obtain a pH of 5.0 were added. Subsequently, 20 mg of fluticason-17-propionate and a sufficient amount of WFI to yield a total weight of 100 g were added. The liquid was stirred at room temperature for about 5 hours. The resulting solution was filtered through a sterile filter having a pore size of 0.22 µm. The assay of the resulting solution showed a content of about 20 µg/ml formoterol and 200 µg/ml fluticason-17-propionate. The composition had a dynamic viscosity of about 1.35 mPas and a surface tension of approx. 60 mN/m. A volume of 0.5 ml, each, of the sterile filtered solution was filled into 0.6 ml blow-fill-seal vials made of low density polyethylene. The plastic vials are packed in aluminium laminate overpouches. Later the entire content of a vial was transferred into the medication cup of the electronic inhaler eFlow for oral inhalation, intended for the treatment of asthma.

The formulation showed increased stability of formoterol (FOR) in comparison to an aqueous formoterol solution without fluticasone and HPGCD, especially at increased temperatures. The following table presents the stability data after storage for 3 months.

| **Time point** | **Storage conditions** | **Formoterol [% of start value]** | | **Total impurities [area%]** | |
|---|---|---|---|---|---|
| | | 20 µg/ml FOR in WFI | 20 µg/ml FOR formulation example 6 | 20 µg/ml FOR in WFI | 20 µg/ml FOR formulation example 6 |
| Start | - | 100.0 | 100.0 | 0.13 | 0.14 |
| 3 months | 5°C | 98.9 | 98.9 | 0.37 | 0.32 |
| | 25°C / 60%r.h. | 94.3 | 98.9 | 2.34 | 1.24 |
| | 40°C / 75%r.h. | 81.7 | 87.8 | 4.89 | 3.49 |

### Example 7: Preparation of a mometasone and thiotropium solution with HPBCD and HPGCD

5 g of HPGCD and 5 g of HPBCD were dissolved in 85 g of WFI. To the solution, 2 mg of thiotropium bromide, 0.9 g of sodium chloride and a sufficient amount of sodium hydroxide solution (0.1 M) or hydrochloric acid (0.1 M), respectively, to obtain a pH of 6.0 were added. Subsequently, 20 mg of mometasone fuorate and a sufficient amount of WFI to yield a total weight of 100 g were added. The liquid was stirred at room temperature for about 5 hours and filtered through a sterile filter having a pore size of 0.22 µm. The assay of the resulting solution showed a content of about 20 µg/ml thiotropium bromide and 250 µg/ml mometasone fuorate. The composition had a dynamic viscosity of about 1.4 mPas and a surface tension of approx. 60 mN/m. A volume of 0.5 ml, each, of the sterile filtered solution was filled into 0.75 ml blow-fill-seal vials made of low density polyethylene. The plastic vials are packed in aluminium laminate overpouches filled with nitrogen. Later the entire content of a vial was transferred into the medication cup of the electronic inhaler PARI eFlow for oral inhalation, intended for the treatment of COPD.

### Example 8: Nebulisation of a budesonide solution with HPBCD

The aqueous solution prepared according to example 1 was nebulised using a PARI eFlow nebuliser and a PARI Breath Simulator. When an adult breathing pattern characterised by a tidal volume of 500 ml, 15 breaths per minute, and an inhalation to exhalation ratio of 50:50 was used, the device delivered about 60 % of a charged drug dose. In the case of a paediatric breathing pattern, i.e a tidal volume of 150 ml, 20 breaths per minute, and an inhalation/exhalation ratio of 40:60, the delivered dose was about 55 % relative to the charged dose.

Laser diffraction measurement of the particles size of the aerosol revealed a mass median diameter of about 3.1 µm with a geometric standard deviation of about 1.5. The respirable fraction of the aerosol, being defined as the weight fraction of particles having a size of less than 5 µm, was about 88 %. The total output rate was from 420 to 460 mg/min, depending on the flow rate.

### Example 9: Stability of a budesonide solution with HPBCD

The aqueous solution prepared according to example 1 was stored for 3 months at 40 °C and 75 % r.h. After storage, an assay was performed to determine the budesonide content, which was found to be 293 µg/ml, i.e. not less than at the beginning of storage. The total impurities were at a level of only about 1 %.

## Claims

1. Aqueous pharmaceutical composition suitable for administration as an aerosol, comprising a poorly soluble corticosteroid and a cyclodextrin, wherein the amount of the cyclodextrin is effective to solubilise the corticosteroid.

2. The composition of claim 1, wherein the poorly soluble corticosteroid is selected from the group consisting of budesonide, fluticasone, mometasone, ciclesonide, including the salts, derivatives, and solvates thereof.

3. The composition of claim 1 or 2, wherein the concentration of the corticosteroid is from about 20 to about 1000 µg/ml and more preferably from about 50 to about 500 µg/ml.

4. The composition of claim 1 or 2 or 3 wherein the corticosteroid is combined with a beta-agonist (e.g. formoterol) or an anticholinergic (e.g. thiotropium).

5. The composition of any of the preceding claims, wherein the concentration of the cyclodextrin is from about 1 to about 150 mg/ml and more preferably about 2.5 to 125 mg/ml.

6. The composition of any of the preceding claims, comprising at least one further excipient, said excipient being preferably selected from pharmaceutically acceptable organic acids, salts of organic acids, inorganic acids, inorganic salts, bases, sugars, sugar alcohols, stabilisers, antioxidants, surfactants, preservatives, and taste masking agents.

7. The composition of any of the preceding claims, being **characterised by** a dynamic viscosity in the range from about 0.8 to about 2 mPas, and preferably from about 0.9 to about 1.3 mPas.

8. The composition of any of the preceding claims, **characterised by** a surface tension in the range from about 30 to about 75 mN/m, and preferably from about 45 to about 73 mN/m.

9. The composition of any of the preceding claims, **characterised by** an osmolality of about 200 to about 600 mOsmol/kg, and preferably of about 220 to about 400 mOsmol/kg.

10. The composition of any of the preceding claims, wherein the unit dose of the corticosteroid is about 100 to 300 µg and the unit dose volume is about 0.25 to about 1 ml.

11. The composition of any of the preceding claims, wherein the poorly soluble corticosteroid is budesonide.

12. The composition of claim 11, wherein the cyclodextrin is γ-cyclodextrin, further **characterised by** a concentration of budesonide from about 50 to about 1000 µg/ml, preferably from about 100 to about 500 µg/ml, and a concentration of γ-cyclodextrin from about 5 to about 30 mg/ml.

13. The composition of claim 11, wherein the cyclodextrin is 2-hydroxypropyl-β-cyclodextrin, further **characterised by** a concentration of budesonide from about 50 to about 1000 µg/ml, preferably from about 200 to about 750 µg/ml, and a concentration of 2-hydroxypropyl-β-cyclodextrin from about 10 to about 100 mg/ml.

14. The composition of claim 11, **characterised by** a pH from about 3.5 to about 6, and preferably from about 4 to about 5.

15. The composition of any of claims 1 to 10, wherein the poorly soluble corticosteroid is fluticasone-17-propionate.

16. The composition of claim 15, wherein the cyclodextrin is γ-cyclodextrin, further **characterised by** a concentration of fluticasone-17-propionate from about 50 to about 500 µg/ml, preferably from about 100 to about 300 µg/ml, and a concentration of γ-cyclodextrin from about 20 to about 75 mg/ml.

17. The composition of claim 15, wherein the cyclodextrin is 2-hydroxypropyl-γ-cyclodextrin, further **characterised by** a concentration of fluticasone-17-propionate from about 50 to about 500 µg/ml, preferably from about 150 to about 350 µg/ml, and a concentration of 2-hydroxypropyl-γ-cyclodextrin of at least about 30 mg/ml.

18. The composition of claim 15, **characterised by** a pH from about 4.5 to about 7.5, and preferably from about 5 to about 7.

19. The composition of any of the preceding claims, comprising a further active agent.

20. The composition of claim 19, wherein at least one of the active agents is selected from the group consisting of anti-inflammatory drugs, such as corticosteroids, non steroidal anti-inflammatory drugs (NSAIDs), Leukotriene-Antagonists, Betasympathomimetics, Anticholinergics, Phosphodiesterase-inhibitors, potassium channel openers, Tachikinin and kinin antagonists, IgE-synthesis inhibitors, endothelinreceptor antagonists, anesthetics, immunomodulators, antiinfectives, interferones, Vasodilatators, Angiotension Converting Enzyme (ACE) inhibitors, Cytostatics, in particular: budesonide, ciclesonide, fluticasone, mometasone, beclomethasone, flunisolide; ibuprofen, ketoprofen, valdecoxib, celecoxib; zileuton, montelukast, pranlukast, roflumilast, cilomilast, zafirlukast; albuterol, formoterol, salmeterole, levalbuterole, terbutaline, pirbuterol; tiotropium, oxitropium, ipratropium; theophyllin, pentoxyphyllin, cilomast, rolipram, amrinone, cilostazol, zardaverine, benzafentrine; cromakalim, levocromakalim, pinacidil; Nolpitantium, Saredutant, Nepadutant, Osanetant; Icatibant; Cromoglicate, nedocromil, glucan-sulfates, suplatast, batimastat, omalizumab; idocaine, prolocaine, mepivacaine, bupivacaine, articaine, cyclosporine, tacrolimus, sirolimus, everolimus, rapamycine, leflunomide, midostaurin, azathioprine; chloroquin, hydroxychlorquine; trimethoprim, tetracycline, doxycycline, ciprofloxacine, moxifloxacine, gatifloxacine, carbapenems, azithromycine, clarithromycine, erythromycine, ketolides, penems, aninoglycosides, tobramyicin, filgrastim, pentamidine, microcidin, clerocidin; metronidazole, ketoconazole, itraconazole, voriconazole, clotrimazole, bifonazole, fluconazole, amphotericine B, natamycine, nystatine, amikacine, aciclovir, famciclovir, valaciclovir, didanosine, saquinavir, ritonavir, lamivudine, stavudine, zidovudine, ribivarine, captoprile, lisinoprile, perindoprile, trandolaprile, cilazaprile, lovastatin, relaxin; suramin; sildenfafil, tardalafil, vardenafil, nitrendipin, amlodipine, prostacyclins, beraprost, Iloprost, bosentane, , carmustine, lomustine, taxol, etopside, cisplatin; reduced glutathione, TNF-alpha-antibodies including the pharmaceutically acceptable derivatives of these substances such as, for example, the salts, conjugates, enantiomeres, racemates, epimeres, diastereomeres or complexes of any one of these active agents.

21. A solid composition adapted for preparing the aqueous pharmaceutical composition of any of claims 1 to 20 by mixing said solid composition with an aqueous carrier.

22. A medicament comprising the composition of any claims 1 to 20 for the treatment of upper and lower respiratory diseases.

23. The medicament of claim 22, being packaged in one or more single dose primary packaging means, from which at least 80% of the nominal dose is dispensable, by dropwise dosing with a dispensing uniformity of less than about 10 % of the mean dispensed dose wherein the packaging means is adapted to hold a fill volume of about 0.2 ml to about 5 ml.

24. The medicament of claim 23, wherein each packaging means is a plastic container comprising a removable closure element, and wherein the container is optionally manufactured using an aseptic blow-fill-seal process design.

25. The medicament of claim 24, wherein the composition is in aqueous liquid form, and wherein the removal of the closure element yields an opening in the container of a diameter approximately corresponding to the inner or outer diameter of a male luer connection.

26. The medicament of 24, wherein the plastic container is designed to be, upon removal of the removable closure elements, tightly connectable to a connecting member of a nebuliser to form a closed system.

27. The medicament of any of claims 24 to 26, wherein the plastic container comprises one or more volume or dosing marks and/or one or more imprints.

28. The medicament of claim 22, being packaged in one or more multiple dose primary packaging means which is adapted to hold a fill volume of about 2 ml to about 50 ml.

29. Method for the preparation of the composition of any of claims 1 to 20, comprising the following sequential steps:
(a) providing the ingredients of the composition;
(b) combining the ingredients provided in step (a) to form an aqueous liquid composition;
(c) sterile filtration of the composition obtained in step (b); and
(d) filling the sterile filtered composition obtained in step (c) into sterile containers under aseptic conditions.

30. The method of claim 29, wherein at least one of the ingredients provided in step (a) is sterile.

31. The method of claim 29 or 30, wherein step (b) comprises a substep of equilibration over a period of at least 1 hour, and preferably at least 3 hours.

32. The method of any of claims 29 to 31, wherein the sterile containers of step (d) are plastic vials manufactured by an aseptic blow-fill-seal process.

33. A method for delivering a pharmaceutical aerosol to a human in need thereof, comprising:
providing an aqueous solution of a poorly soluble corticosteroid and a cyclodextrin in an amount effective to solubilise and stabilise the corticosteroid or its combination drug;
providing a nebuliser adapted to nebulise said aqueous solution into an aerosol having a mass median aerodynamic diameter of about 1 to about 5 µm, and more particularly of about 1.5 to about 4 µm, and
using said nebuliser to nebulise said aqueous solution.

34. The method of claim 33, wherein the nebuliser is adapted to deliver the aerosol at a total output rate of at least about 250 mg/min, and preferably of at least 350 mg/min with a delivered dose ex mouthpiece greater than 30% and more preferably greater than 50% of the nominal dose based on a standard sinusoidal breathing pattern of 15 breaths of 500 ml/min and an inhalation to exhalation ratio of 1:1.

35. The method of claim 33 or 34, wherein the aerosol exhibits a mass median aerodynamic diameter from about 2 to about 4 µm with a geometric standard deviation from about 1.3 to about 2.5.

36. The method of claim 33 or 34 or 35, wherein the nebuliser is a jet nebuliser.

37. The method of claim 36, wherein the jet nebuliser comprises a nose piece for directing the aerosol into one or both nostrils of a patient and is capable of generating a vibrating aerosol, such as the PARI Sinus nebuliser.

38. The method of any of claims 33 to 37, wherein said human suffers from one or more diseases, symptoms or conditions affecting the mucosa of the nasal cavity and/or a paranasal sinus, such as an acute, subacute or chronic form of allergic rhinitis, vasomotoric rhinitis, seasonal rhinitis, perennial rhinitis, chronic rhinitis, rhinosinusitis, sinusitis, or nasal polyps.

39. The method of any of claims 33 to 38, wherein the nebuliser is an electronic nebuliser operating with a vibrating membrane with pores of defined size, such as an eFlow®, eFlow® rapid, AeroNeb®, AeroDose®, AERx®, MicroAir® or ODEM device.

40. The method of any of claims 33 to 37, wherein said human suffers from one or more diseases, symptoms or conditions selected from the group consisting of asthma, paediatric asthma, allergic and viral sinusitis, rhinosinusitis, nose and paranasal cavity infections, obstructive bronchitis, chronic obstructive bronchitis, chronic obstructive pulmonary disease (COPD), emphysema, lung infections, vascular, parenchymal lung disease, sarcosidosis, pulmonary fibrosis, cystic fibrosis, bonchiolitis obliterans, and lung cancer.

41. The method of any of claims 33 to 40, wherein the aqueous solution is provided at a volume in the range from about 0.2 to about 5 ml, and in particular from about 0.5 to about 1.0 ml

42. The method of any of claims 33 to 41, wherein the nebuliser is adapted to nebulise said volume of the aqueous solution within about 5 minutes or less, and in particular within about 2 minutes or less.
